# EUROPEAN PATENT APPLICATION

(11) **EP 3 112 335 A1**
(43) Date of publication of application: **04.01.2017**
(21) Application number: 15754741.5
(22) Date of filing: 20.02.2015
(51) Int. Cl.: C07C 5/10, C07B 61/00, C07C 1/10, C07C 9/04, C07C 13/18

(54) **AROMATIC COMPOUND HYDROGENATION SYSTEM AND HYDROGENATION METHOD**

(30) Priority: 25.02.2014 JP 2014034048
(71) Applicant: Chiyoda Corporation, Kanagawa 220-8765 (JP)
(72) Inventor: KAWAI, Hironori, Yokohama-shi Kanagawa 220-8765 (JP); SHIRAGA, Masato, Yokohama-shi Kanagawa 220-8765 (JP); IMAGAWA, Kenichi, Yokohama-shi Kanagawa 220-8765 (JP); ISHIYAMA, Tatsuo, Yokohama-shi Kanagawa 221-0022 (JP)
(74) Representative: Matthews, Derek Peter
(86) International application number: PCT/JP2015/000831
(87) International publication number: WO 2015/129228

(57) **Abstract**

In a system for hydrogenation of an aromatic compound, an excessive temperature rise in the hydrogenation reaction unit is prevented, and the amount of the dilution gas to be circulated is minimized. The hydrogenation system (1) comprises a hydrogenation reaction unit (2) for producing a hydrogenated aromatic compound by adding hydrogen to an aromatic compound via a hydrogenation reaction, a separation unit (3) for separating the hydrogenated aromatic compound from a product of the hydrogenation reaction unit, and a transportation unit (4) for circulating at least a part of a residual component remaining in the separation unit after separating the hydrogenated aromatic compound therefrom to the hydrogenation reaction unit. The hydrogen supplied to the hydrogenation reaction unit consists of diluted hydrogen (L2) diluted by a dilution compound having a higher molar specific heat than nitrogen, and the dilution compound includes a component circulated to the hydrogenation reaction unit as the residual component.

## Description

### TECHNICAL FIELD

The present invention relates to a system and a method for hydrogenating an aromatic compound, and in particular to a system and a method for hydrogenating an aromatic compound suitable for use in an organic chemical hydride process for storing and transporting hydrogen in the form of a hydrogenated aromatic compound obtained by hydrogenating an aromatic compound.

### BACKGROUND ART

The organic chemical hydride process for hydrogenating aromatic compounds such as toluene has recently been developed for the purposes of storing and transporting hydrogen in the form of hydrogenated aromatic compounds (or organic hydrides). According to this process, hydrogen is converted into a hydrogenated aromatic compound at the site of hydrogen production, and transported in the form of the hydrogenated aromatic compound. The hydrogenated aromatic compound is separated into the hydrogen and the aromatic compound by dehydrogenating the hydrogenated aromatic compound at a plant or a hydrogen station located near a city or other user of hydrogen. The aromatic compound produced from this dehydrogenation process is transported back to the production site of hydrogen to be hydrogenated by hydrogen once again.

Because the hydrogenation of an aromatic compound is an exothermic reaction, the reaction heat may raise the temperature of the reaction unit (or vessel) to such an extent that the catalyst may be degenerated and an undesired reaction may be caused. It is therefore known to control an excessive rise in the temperature of the reaction unit by diluting the hydrogen that is used for the reaction by using an inert dilution gas such as nitrogen (See Patent Document 1).

### PRIOR ART DOCUMENT(S)

### PATENT DOCUMENT(S)

[Patent Document 1] JP2011-207641A

### SUMMARY OF THE INVENTION

### TASKS TO BE ACCOMPLISHED BY THE INVENTION

The prior art disclosed in Patent Document 1 allows an excessive rise in the temperature of the hydrogenation reaction unit caused by the hydrogenation reaction, but owing to the significant increase in the mass of the reactant and/or the product caused by the use of the dilution gas, the size of the hydrogenation reaction unit and the associated equipment is required to be increased, and this causes the equipment cost to be significantly greater than that required when the hydrogen is not diluted.

With the aim of improving the process efficiency in a hydrogenation system for an aromatic compound, the inventors of this application have conceived the idea of circulating the unreacted substances (such as dilution gas and residual hydrogen) other than the main product (hydrogenated aromatic compound) of the hydrogenation reaction to the hydrogenation reaction unit. When implementing such an idea into an actual structure, it is desired that the size of the hydrogenation reaction unit and the associated equipment is minimized, and the amount of the unreacted substances (in particular the amount of the dilution gas) is also reduced so that the power consumption by the transportation unit (such as a compressor) required for the circulation is minimized.

The present invention was made in view such a problem of the prior art, and has a primary object to provide a system and a method for hydrogenating an aromatic compound involving the use of a structure for circulating at least part of the dilution compound for diluting the hydrogen that is used for the hydrogenation reaction that prevents an excessive temperature rise in the hydrogenation reaction unit and minimizes the amount of the dilution compound to be circulated.

### MEANS TO ACCOMPLISH THE TASK

According to a first aspect of the present invention, the present invention provides a system for hydrogenating an aromatic compound, comprising: a hydrogenation reaction unit (2) for producing a hydrogenated aromatic compound by adding hydrogen to an aromatic compound via a hydrogenation reaction; a separation unit (3) for separating the hydrogenated aromatic compound from a product of the hydrogenation reaction unit; and a transportation unit (4) for circulating at least a part of a residual component remaining in the separation unit after separating the hydrogenated aromatic compound therefrom to the hydrogenation reaction unit; wherein the hydrogen supplied to the hydrogenation reaction unit consists of diluted hydrogen (L2) diluted by a dilution compound having a higher molar specific heat than nitrogen, and the dilution compound includes a component circulated to the hydrogenation reaction unit as the residual component.

In the hydrogenation system for an aromatic compound provided by the first aspect of the present invention, when circulating at least a part of the dilution compound for diluting the hydrogen that is to be used for the hydrogenation reaction, the concentration of the hydrogen is appropriately adjusted by selecting the amount of the dilution compound so that an excessive temperature rise in the hydrogenation reaction unit is prevented and the amount of the dilution compound to be circulated is minimized. Thereby, the size of the hydrogenation reaction unit and the associated equipment can be minimized, and the power that is consumed by the transportation unit for the circulation can be minimized.

According to a second aspect of the present invention, in conjunction with the first aspect of the present invention, the dilution compound in the diluted hydrogen is in a concentration equal to or greater than 10 vol% and less than 30 vol%.

The hydrogenation system for an aromatic compound provided by the second aspect of the present invention allows excessive temperature rise in the hydrogenation reaction unit to be controlled and the amount of the circulating dilution compound to be minimized by maintaining the concentration of the dilution compound within an appropriate range. More specifically, by maintaining the concentration of the dilution compound within the range mentioned above, any rapid increase in the temperature of the hydrogenation reaction can be controlled, and the hydrogenation reaction unit and the associated equipment can be prevented from becoming undesirably bulky so that the space and energy requirements of the hydrogenation system can be minimized.

According to a third aspect of the present invention, in conjunction with the first aspect or the second aspect of the present invention, the system further comprises a circulation line (L6) for transporting the residual component to the hydrogenation reaction unit, wherein the circulation line is connected to an expulsion line (L7) for expelling a part of the residual component to outside.

The hydrogenation system for an aromatic compound provided by the third aspect of the present invention allows the concentration of the dilution compound in the diluted hydrogen and the pressure of the hydrogenation system to be adjusted by allowing a part of the residual component to be expelled to outside. For instance, the pressure adjustment of the hydrogenation system may take the form of adjusting the pressure of the hydrogenation reaction unit for the benefit of allowing the reaction condition of the hydrogenation reaction unit to be changed.

According to a fourth aspect of the present invention, in conjunction with any one the first to third aspects of the present invention, the dilution compound includes methane, and the system further comprises a reformer unit (41) for producing reformer gas containing hydrogen and carbon monoxide from the methane via a steam reforming process, and a methanation reaction unit (43) for producing methane from the carbon monoxide produced by the reformer unit via a methanation reaction, the hydrogen produced by the reformer unit and the methane produced by the methanation reaction unit being supplied to the hydrogenation reaction unit at least as a part of the diluted hydrogen.

The hydrogenation system for an aromatic compound provided by the fourth aspect of the present invention allows hydrogen to be efficiently obtained by using methane which is a main component of natural gas and abundantly available, and the methane remaining in the obtained hydrogen to be used for the dilution compound.

A fifth aspect of the present invention provides a method for hydrogenating an aromatic compound, comprising: a hydrogenation reaction step for producing a hydrogenated aromatic compound by adding hydrogen to an aromatic compound via a hydrogenation reaction, a separation step for separating the hydrogenated aromatic compound from a product of the hydrogenation reaction step; and a transportation step for circulating at least a part of a residual component remaining after the separation step separating the hydrogenated aromatic compound therefrom for use in the hydrogenation reaction step; wherein the hydrogen supplied for the hydrogenation reaction step consists of diluted hydrogen diluted by a dilution compound, and the dilution compound includes a component circulated to the hydrogenation reaction step as the residual component.

According to the present invention, in an arrangement where at least a part of the dilution compound for diluting the hydrogen used for the hydrogenation reaction is circulated to the hydrogenation reaction unit, excessive rise in the temperature of the hydrogenation reaction unit can be controlled, and the amount of the dilution compound that is to be circulated can be minimized.

### BRIEF DESCRIPTION OF THE DRAWINGS

Figure 1 is a block diagram showing the simplified overall structure of a hydrogenation system for an aromatic compound embodying the present invention;
Figure 2 is a graph showing the influences of the dilution ratio of the dilution gas in the hydrogen on the equilibrium temperature and the equilibrium conversion ratio;
Figure 3 is a graph showing the relationship between the concentration of the dilution gas in the reaction gas and the reduction in the reaction temperature (peak value) obtained experimentally; and
Figure 4 is a block diagram of a hydrogen production system for producing hydrogen (diluted hydrogen) supplied to the hydrogen reaction unit shown in Figure 1.

### PREFERRED EMBODIMENT(S)

An embodiment of the present invention is described in the following with reference to the appended drawings.

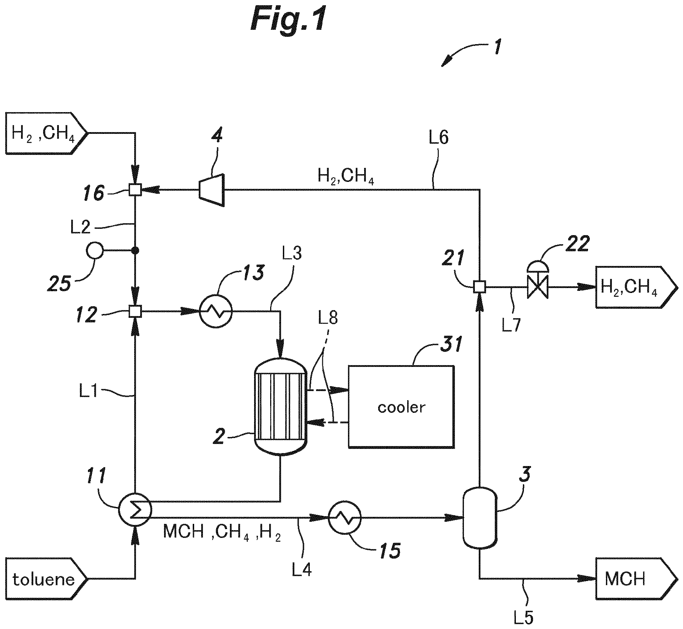

Figure 1 is a block diagram showing the simplified overall structure of a hydrogenation system 1 for an aromatic compound embodying the present invention. The hydrogenation system 1 produces a hydrogenated aromatic compound (consisting of methylcyclohexane in this case) by adding hydrogen to an aromatic compound (consisting of toluene in this case) for the purposes of storing and transporting hydrogen.

The hydrogenation system 1 essentially consists of a hydrogenation reaction unit 2 for producing methylcyclohexane (MCH) by adding hydrogen to toluene in a hydrogenation reaction, a separation unit 3 for separating MCH from the product of the hydrogenation reaction unit 2 and a transportation unit 4 for circulating at least a part of the residual component which remains after the MCH (main product) has been separated by the separation unit 3.

In this hydrogenation system 1, toluene is supplied via an aromatic compound supply line L1 as a reactant of the hydrogenation reaction. The aromatic compound supply line L1 is provided with a preheater 11 for preheating the toluene by exchanging heat with the product of the hydrogenation reaction as will be discussed hereinafter. The hydrogenation system 1 also receives hydrogen via a hydrogen supply line L2 as another reactant of the hydrogenation reaction. In this case, the hydrogen that is supplied here consists of diluted hydrogen diluted by methane. In other words, the hydrogen that is to be used for the hydrogenation reaction is mixed with methane in advance (or the hydrogen is diluted by the methane) so that the concentration of the hydrogen is appropriately adjusted. Alternatively, the hydrogen and the methane may be individually introduced into the hydrogenation system 1, and mixed therein.

The toluene and the diluted hydrogen are mixed in a merging point 12, and then introduced into the hydrogenation reaction unit 2 via a material supply line L3. The toluene and the diluted hydrogen are preheated to a prescribed temperature in a preheater 13 provided in the material supply line L3. The preheaters 11 and 13 may consist of any per se known arrangements which are capable of preheating the toluene and the diluted hydrogen to the necessary extent.

In the hydrogenation reaction unit 2, MCH is produced by the hydrogenation reaction between the toluene and the hydrogen (hydrogenation reaction process). The product (mainly consisting of MCH, and containing small amounts of methane and unreacted residual hydrogen) of the hydrogenation reaction unit 2 is forwarded to a separation unit 3 via a product transportation line L4. The product transportation line L4 is connected to the preheater 11 so that the product is cooled by exchanging heat with the toluene. A cooler 15 is provided in the product transportation line L4 downstream of the preheater 11 to further cool the product to a prescribed temperature required to liquefy the MCH. The first cooler 15 may consist of any per se known cooler as long as it is capable of cooling the product as required (liquefaction of the MCH in this case).

The product of the hydrogenation reaction is separated into a gas and a liquid component in the separation unit 3 consisting of a per se known gas-liquid separator (separation step). The separation unit 3 separates the MCH (containing the unreacted toluene) as the liquid component and the residual component (gas such as methane, unreacted hydrogen and byproducts) as the gas component.

The separated MCH is recovered via a MCH recovery line L5, and is stored in a storage tank not shown in the drawings. The separated residual component (gas such as methane, unreacted hydrogen and byproducts) is forwarded to a merging point 16 of the hydrogen supply line L2 via a residual gas circulation line L6 to be mixed with the hydrogen (transportation step). Thereby, the methane serving as the dilution gas is circulated to the hydrogenation reaction unit 2. The circulation of the residual component is carried out by a transportation unit 4 provided in the residual gas circulation line L6. The transportation unit 4 may consist of a per se known compressor for pressurization, but may also consist of any other per se known arrangement providing a similar function.

A gas expulsion unit 21 provided in the residual gas circulation line L6 is connected to a gas expulsion line L7. In this hydrogenation system 1, the pressure within the hydrogenation system 1 can be adjusted by controlling a regulator valve 22 provided in the gas expulsion line L7 to expel a part of the residual components to the outside. The pressure adjustment of the hydrogenation system 1, for instance by the adjustment of the pressure in the circulation line, allows the transportation system to be operated in a stable manner. The pressure adjustment of the hydrogenation system 1 also allows the reaction condition to be controlled, and the separation condition for the gas-liquid separation to be controlled.

The hydrogen supply line L2 is provided with a hydrogen concentration detector 25, and the concentration of the diluted hydrogen (or the concentration of methane) can be easily adjusted to a prescribed range by controlling the opening and closing of the regulator valve 22 according to the detection result of the hydrogen concentration detector 25. The regulator valve 22 may be controlled in such a manner that, for instance, when the hydrogen concentration of the diluted hydrogen supplied to the hydrogenation system 1 is substantially constant (or when the amount of the supplied methane is constant), a substantially same amount of methane as the amount of the supplied methane is expelled from the gas expulsion line L7. The methane expelled from the gas expulsion line L7 can be reused as the dilution gas for the hydrogen so that the need for the supply of fresh methane to the hydrogenation system 1 may be substantially eliminated. The byproducts of the hydrogenation reaction remaining in the residual component can be removed by the expulsion of the residual component by the regulator valve 22.

The hydrogenation reaction unit 2 consists of a per se known heat exchanger type, multitubular fixed bed reactor not shown in the drawings, and includes a plurality of reaction tubes filled with hydrogenation catalyst (solid catalyst) and received in a shell. As will be discussed hereinafter, because the hydrogenation reaction is an exothermic reaction, the reaction heat must be removed so that the drop in the conversion ratio due to a rise in the reaction temperature may be avoided. The shell of the multitubular fixed bed reactor is provided with a cooling jacket having a heat medium (such as oil, water and steam) circulating therein. The heat medium which is heated by the reaction heat of the hydrogenation reaction is forwarded to a cooler 31 via a heat medium circulation line L8, and after being cooled therein, circulated back to the multitubular fixed bed reactor via the heat medium circulation line L8. The reaction temperature of the hydrogenation reaction is appropriately adjusted by the cooling arrangement for the hydrogenation reaction unit 2. When the heat medium expelled from the heat medium circulation line L8 consists of steam, the heated steam can be industrially used for heating and humidifying purposes as well as for powering purposes.

The structure of the hydrogenation reaction unit 2 is not limited to that of the illustrated embodiment, but may also take other forms as long as the required hydrogenation reaction according to the present invention can be achieved. Although not described in any detail, the various lines L1 to L8 in the hydrogenation system 1 may consist of per se known structures including piping, valves and pumps.

In the hydrogenation reaction unit 2, hydrogen is chemically added to toluene (C₇H₈) by the hydrogenation reaction that can be represented by Chemical Equation (1) given in the following. This hydrogenation reaction is an exothermic reaction (ΔH₂₉₈ = -205 kJ/mol), and the toluene is converted into MCH (C₇H₁₄).

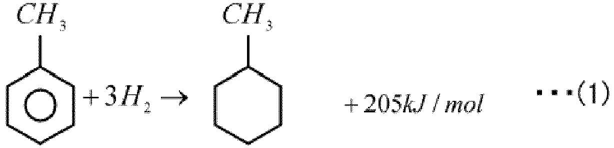

The reaction temperature for this hydrogenation reaction may be in the range of 150°C to 250°C, or more preferably in the range of 160°C to 220°C. The amount of the byproducts (the amount produced by the hydrogenation reaction) remaining in the residual component separated from the MCH in the separation unit 3 can be controlled by limiting the rise in the reaction temperature of the hydrogenation reaction. The reaction pressure for this hydrogenation reaction may be in the range of 0.1 MPaG to 5 MPaG, or more preferably in the range of 0.5 MPaG to 3 MPaG. The supply ratio of toluene and hydrogen (toluene/hydrogen mols) to the hydrogenation reaction unit 2 should be stoichiometrically 1/3, but may be in the range of 1/2 to 1/10, or more preferably in the range of 1/2.5 to 1/5, or most preferably in the range of 1/3 to 1/4. A full reaction of toluene can be achieved by optimizing the lower limit of the supply ratio of toluene and hydrogen to the hydrogenation reaction unit 2, and the amount of the gas component that is supplied to the hydrogenation system 1 is prevented from becoming excessive so that the power requirement of the transportation unit 4 can be minimized by optimizing the upper limit of the supply ratio. The excess amount of hydrogen for the given amount of toluene will remain in the residual component of the hydrogenation reaction so that the toluene can be fully hydrogenated, and the surplus of hydrogen can be recycled for the subsequent hydrogenation reaction.

The concentration of methane in the diluted hydrogen (including the hydrogen and the methane in the residual component from the previous reaction if the residual component is mixed therewith) for the hydrogenation reaction may be in the range of 5 vol% to 70 vol%, or more preferably in the range of 10 vol% to 30 vol%. In this case, the concentration of hydrogen (which is diluted by methane) may be in the range of 30 vol% to 95 vol%, or more preferably in the range of 70 vol% to 90 vol%.

In this hydrogenation system 1, by limiting the concentration of methane in the diluted hydrogen supplied as a reactant to be below 30 vol%, not only a rapid rise in the temperature of the hydrogenation reaction unit can be avoided but also the size of the hydrogenation reaction unit 2 and the associated equipment can be minimized so that the space and energy requirements of the hydrogenation system 1 can be minimized. In particular, in this hydrogenation system 1, if the concentration of methane in the diluted hydrogen supplied as a reactant is maintained above 10 vol%, a sufficient amount of circulation can be maintained so that the pressure in the hydrogenation system 1 can be controlled in a stable manner while the benefits of the dilution using methane can be effectively ensured at the same time.

Because there is no residual component immediately following the startup of the hydrogenation system 1, the reactant gas for the hydrogenation reaction at such a time consists solely of diluted hydrogen which has been freshly introduced into the hydrogenation system. On the other hand, when the hydrogenation system is operating under a steady state condition, the residual component is mixed with the diluted hydrogen at the merging point 16 of the hydrogen supply line L2. As the residual component mainly consists of methane, the methane for dilution can be partly replenished by the residual component (circulation gas).

As discussed above, in this hydrogenation system 1, because at least a part of the dilution gas for diluting the hydrogen for the hydrogenation reaction is circulated back to the hydrogenation reaction unit 2, and the concentration of the hydrogen is controlled by the dilution with methane, an excessive rise in the reaction temperature of the hydrogenation reaction that may occur in spite of the cooling in the cooler 31 can be avoided while minimizing the amount of the circulated residual component.

Because methane that is used for diluting the hydrogen has a higher molar specific heat than conventional dilution gas (such as nitrogen), an excessive rise in the temperature of the hydrogenation reaction in the hydrogenation reaction unit 2 can be effectively controlled. For instance, under the condition of a pressure of 0.1 MPa and a temperature of 200 °C, whereas the isobaric specific heat Cp and the isobaric molar specific heat of nitrogen are about 1.05 kJ/kg·K and 29.5 J/mol·K, respectively, the isobaric specific heat Cp and the isobaric molar specific heat of methane are about 2.73 kJ/kg·K and 44.7 J/mol·K, respectively. Furthermore, because methane has a higher heat conductivity than conventional dilution gas (such as nitrogen), the removal of heat from the hydrogenation reaction is promoted so that the cooling efficiency of the cooler 31 via the cooling jacket can be enhanced. For instance, at a temperature of 200 °C, whereas the thermal conductivity of nitrogen is about 0.038 W/m·K, the thermal conductivity of methane is about 0.05W/m·K which is significantly higher than that of nitrogen.

The hydrogen used for the hydrogenation reaction was diluted only by methane in the illustrated embodiment for the convenience of description, but the dilution gas may also contain compounds (such as lower hydrocarbons other than methane) other than methane as long as the concentration of such compounds is low enough not to obstruct the dilution effect of methane according to the present invention. The manufacturing methods and the mining methods for the hydrogen and the methane that are supplied to the hydrogenation system 1 as reactants are not limited, but may consist of any other methods. For instance, the methane may be obtained from natural gas, biomethanation gas, methane hydrate, shale gas and utility gas.

The dilution gas that can be used for the present invention is not limited to methane, but may also consist of any other compounds (dilution compounds) that have a higher molar specific heat than nitrogen under the temperature and pressure condition of the hydrogenation reaction (a molar specific heat higher than 30 J/mol·K under the condition of a pressure of 0.1 MPa and a temperature of 200 °C). For instance, such dilution compounds may be selected from steam having a molar specific heat of about 35.8 J/mol·K, ethane having a molar specific heat of about 76 J/mol·K, propane having a molar specific heat of about 117 J/mol·K, and carbon dioxide having a molar specific heat of about 43.8 J/mol·K, all under the condition of a pressure of 0.1 MPa and a temperature of 200 °C. The thermal conductivity of the dilution compound (including mixtures) is preferred to be higher than that of nitrogen under the temperature and pressure condition of the hydrogenation reaction. The hydrocarbons that can be used for the dilution compounds preferably consist of saturated hydrocarbons having a carbon number of seven or less. The saturated hydrocarbons may be either cyclic or non-cyclic, but preferably consist of low activity hydrocarbons without any functional groups. The dilution compound may be in the form of a mixture of two or more gases, and the mixing ratio may be suitably adjusted so that the desired molar specific heat and/or the desired thermal conductivity may be achieved.

The aromatic compound that may be used for the hydrogenation reaction in the hydrogenation reaction unit 2 is not limited to toluene, but may also consist of a monocyclic aromatic compound such as benzene and xylene, a bicyclic aromatic compound such as naphthalene, tetralin and methylnaphthalene, a tricyclic aromatic compound such as anthracene, or a combination of two or more of such aromatic compounds.

The hydrogenated aromatic compound which is the main product of the hydrogenation reaction is produced by hydrogenating any of the aforementioned aromatic compounds, and may consist of a monocyclic hydrogenated aromatic compound such as cyclohexane and methylcyclohexane, a bicyclic hydrogenated aromatic compound such as tetralin, decaline and methyldecaline, a tricyclic hydrogenated aromatic compound such as tetradecahydroanthracene, or a combination of two or more of such hydrogenated aromatic compounds. Such organic hydrides should be selected from those that are in a stable liquid form under a normal pressure and temperature condition for the convenience of transportation and storage.

The hydrogenation catalyst may consist of at least one of active metals such as platinum (Pt), palladium (Pd) and nickel (Ni) carried by a carrier consisting of alumina, or silica, but may also consist of any other per se known catalyst used for hydrogenating an aromatic compound.

The hydrogenation system 1 may form a part of a storage and transportation system for hydrogen (not shown in the drawings), and the method for storage of organic hydride and production of hydrogen from the organic hydride may be based on the organic chemical hydride process.

For the details of the organic chemical hydride method, reference may be made to "Development of Dehydrogenation Catalyst for Organic Chemical Hydride Method" by Yoshimi OKADA, et al., Catalysts & Catalysis, 2004, 46 (6), p510-512, ISSN 05598958, "Dehydrogenation Catalyst Development for Organic Chemical Hydride Method and Hydrogen Energy Chain Vision", by Yoshimi OKADA, et al., Catalysts & Catalysis, 2009, 51 (6), p496-498 , ISSN 05598958, "Development of Dehydrogenation Catalyst for Organic Chemical Hydride Method with the Aim to Establish a Mass, Long-Distance Storage and Transportation Technology of Hydrogen Energy" by Yoshimi OKADA, et al., Chemical Engineering, 2010, 74 (9), p468-470, ISSN 03759253, and "Development of Dehydrogenation Catalyst for Organic Chemical Hydride Method in Hydrogen Storage and Transportation (New Year Special Edition, GCS Symposium 2005)", by Yoshimi OKADA, et al., Fine Chemical, 2006, 35 (1), p5-13, in lieu of a detailed discussion in this application.

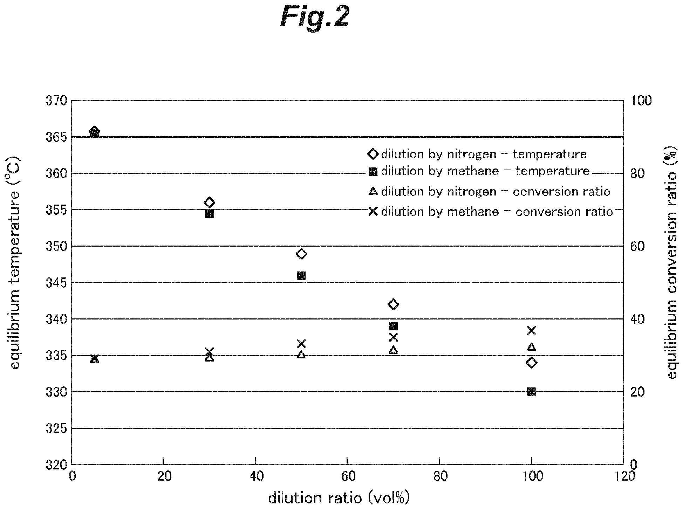

Figure 2 is a graph showing the influences of the dilution ratio of the dilution gas in the hydrogen on the equilibrium temperature and the equilibrium conversion ratio (computation results). This is based the theoretical computations on the hydrogenation reaction of toluene which is performed as an adiabatic reaction with the supply ratio (toluene/hydrogen molar ratio) of toluene and hydrogen to the hydrogenation reaction unit at 1/3.5 under the condition of a pressure of 1.1 MPa and a temperature of 200 °C. Methane and nitrogen (for comparison) were selected as the dilution gas for diluting hydrogen, and the abscissa of the graph represents the dilution ratio (vol%) of methane with the hydrogen serving as the material given as 100 vol%.

In Figure 2, the equilibrium temperature in the hydrogenation reaction lowers with an increase in the dilution ratio (or the decrease in the hydrogen concentration in the reaction gas), but the dilution by methane has a more pronounced effect on the lowering of the equilibrium temperature than the dilution by nitrogen. Therefore, the equilibrium conversion ratio in the hydrogenation reaction is greater when the dilution is made by methane than when the dilution is made by nitrogen.

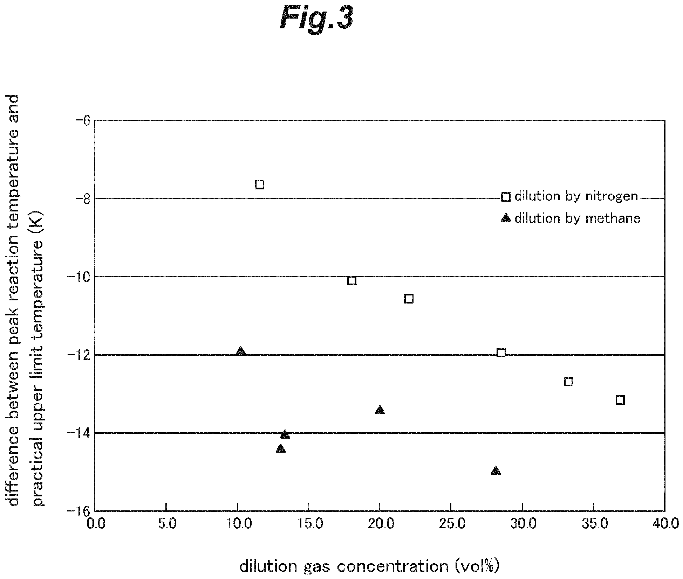

Figure 3 is a graph showing the relationship between the concentration of the dilution gas in the reaction gas and the reduction in the reaction temperature (peak value) obtained experimentally. In this experiment, the hydrogenation reaction of toluene is performed in a heat exchanger type, tubular-flow reactor. The hydrogenation reaction was performed with the supply ratio (toluene/hydrogen molar ratio) of toluene and hydrogen to the hydrogenation reaction unit at 1/3.5 under the condition of a temperature of 150 °C, a pressure of 0.45 MPa and a LHSV (liquid hourly space velocity) of 1.1 /h. Methane and nitrogen (for comparison) were selected as the dilution gas for diluting hydrogen, and the abscissa of the graph represents the concentration (vol%) of the dilution gas in the reaction gas (diluted hydrogen).

In Figure 3, the difference between the peak reaction temperature of the hydrogenation reaction and the practical upper limit temperature (190 °C in this case) decreases (or the peak reaction temperature lowers) with an increase in the concentration of the dilution gas (or a decrease in the hydrogen concentration in the reaction gas). In particular, when the concentration of the dilution gas is in the range of 10 vol% to 30 vol%, the methane is more effective in lowering the peak reaction temperature than nitrogen when used as a dilution gas for the hydrogen. For instance, when the hydrogen is diluted by methane with a methane concentration of about 10 vol%, the difference between the peak reaction temperature of the hydrogenation reaction and the practical upper limit temperature is -12 °C. If a similar result is to be obtained by using nitrogen for the dilution gas, the nitrogen concentration has to be raised to about 28 vol%.

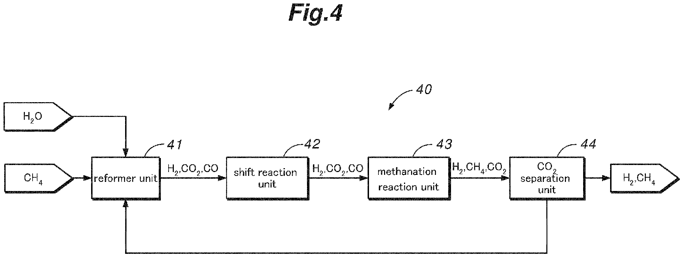

Figure 4 is a block diagram of a hydrogen production system 40 for producing hydrogen supplied to the hydrogen reaction unit shown in Figure 1. The hydrogen production system 40 comprises a reformer unit 41 for producing reformer gas containing hydrogen and carbon monoxide by steam reforming of methane, a water-gas shift reaction unit 42 for producing shift gas containing carbon dioxide and hydrogen by shifting the carbon monoxide gas in the reformer gas to carbon dioxide (shift reaction), a methanation reaction unit 43 for producing methane by a methanation reaction of the carbon monoxide (residual carbon monoxide remaining after the shift reaction) produced by the reformer unit 41, and a carbon dioxide (CO₂) separation unit 44 for separating carbon dioxide from the product gas of the methanation reaction. The hydrogen production system 40 may be formed as a part of the hydrogenation system 1.

In the reformer unit 41, a reforming reaction is carried out by reacting methane with steam at a high temperature (500 °C - 900 °C, for instance) in the presence of catalyst (such as nickel catalyst). The steam reforming process is mainly based on a reaction that can be represented by Chemical Equations (2) and (3) given below.

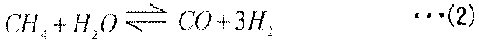

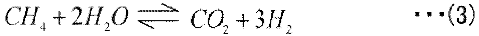

In the shift reaction unit 42, the carbon monoxide in the reformer gas is shifted to carbon dioxide under a prescribed temperature condition in the presence of catalyst to increase the concentration of hydrogen in the reformer gas produced from the reformer unit 41. The reaction temperature of the shift reaction unit 42 can be appropriately selected by taking into account the reaction speed of the shift reaction and the composition ratio of the product. In this case, the concentration of carbon monoxide is reduced in two stages consisting of a shift reaction of a relatively high temperature (about 350 °C - 420 °C) and a shift reaction of a relatively low temperature (about 200 °C - 300 °C). The shift reaction can be mainly represented by Chemical Equation (4) given in the following.

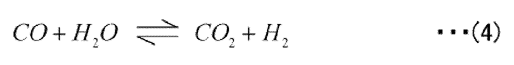

The steam reforming process may be based on per se known technology such as the ICI process (Imperial Chemical Industries, Ltd.) and the Topsoe process (Halder Topsoe). The partial oxidation reforming process may also be used for converting methane into gas essentially consisting of hydrogen and carbon monoxide in the reformer unit 41. The main reaction of the partial oxidation reforming can be represented by Chemical Equation (5) given in the following.

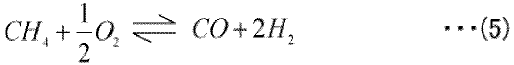

The reforming process may also use the CO₂ conversion process represented by Chemical Equation (6) given in the following in addition to the steam reforming process.

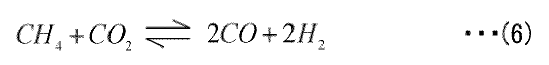

In the methanation reaction unit 43, methane is produced by the methanation reaction performed in the presence of catalyst using the shift gas (hydrogen, carbon monoxide and carbon dioxide) produced by the shift reaction unit 42.

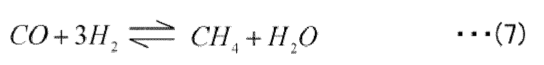

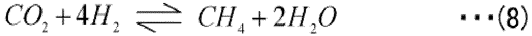

In Chemical Equations (7) and (8), heat is produced and the mol number decreases as the reaction progresses from the left hand side to the right hand side so that the reaction from the left hand side to the right hand side is promoted by lowering the temperature and increasing the pressure. This reaction is an equilibrium reaction, and the product includes methane hydrogen and carbon dioxide. The water produced as a result of the methanation reaction is separated from the methane, hydrogen and carbon dioxide, and expelled from the methanation reaction unit 43 to outside.

The methane and hydrogen produced in the methanation reaction unit 43 is supplied to the carbon dioxide separation unit 44. The carbon dioxide separation unit 44 separates carbon dioxide from hydrogen and methane. The separated hydrogen and methane are used in the hydrogenation process in the hydrogenation system 1 described above. The separated carbon dioxide is forwarded to the reformer unit 41 to be used for the CO₂ reforming process therein.

The carbon dioxide separation unit 44 uses a zeolitic permeation membrane having a highly selective permeability for carbon dioxide as a separation membrane. The zeolitic membrane is prepared by forming a hydrophilic zeolitic film on a porous support member such as alumina and silica. The hydrophilic zeolitic membrane is heat treated at a temperature of 100 °C to 800 °C. The permeation membrane that can be used in the carbon dioxide separation unit 44 is not limited to the zeolitic permeation membrane, but may also consist of other inorganic membranes such as aluminum oxide (alumina) membrane and zirconium oxide (zirconia) membrane.

The CO₂ permeation membrane used in the carbon dioxide separation unit 44 is not limited to the zeolitic permeation membrane mentioned above, but may also consist of other inorganic membrane such as ceramic membrane. The inorganic material in this case preferably consists of ceramic material such as aluminum oxide (alumina) and zirconium oxide (zirconia). If desired, organic membranes (such as high polymer membranes) made of organic materials may also be used. Such organic materials may include cellulose acetate, polysulfone, polyethylene, polypropylene and polyacrylonitrile.

The separation of carbon dioxide in the carbon dioxide separation unit 44 is not necessarily required to be performed by the membrane separation method, but may also be performed by any other per se known separation method such as chemical absorption methods (that cause carbon dioxide to be absorbed via a reaction with alkaline solution such as amines and aqueous solution of potassium carbonate) and physical adsorption methods (that cause carbon dioxide to be adsorbed by contact with an adsorbing agent such as zeolite).

The hydrogen production system 40 may be provided in the hydrogenation system 1, but may also be constructed in a remote location to supply the product (hydrogen and methane) to the hydrogenation system 1 by using a per se known means of transportation (such as ships, trucks and pipelines). The methane that is used as the material for the hydrogen production system 40 may be derived from the methane in the residual component expelled from the gas expulsion line L7 described above. When using the hydrogen and methane separated in the carbon dioxide separation unit 44 in the hydrogenation system 1, the residual component expelled from the gas expulsion line L7 may be mixed therewith.

The present invention has been described in terms of the concrete embodiment thereof which was given only as an example, and should not be interpreted as limiting the present invention. The various components of the hydrogenation system and the hydrogenation method for an aromatic compound according to the present invention discussed above can be partly substituted and omitted without departing from the spirit of the present invention. Also, some of the various components of the embodiment discussed above may be combined into a composite unit combining a plurality of functionalities.

### [List of the Numerals]

- 1: hydrogenation system
- 2: hydrogenation reaction unit
- 3: separation unit
- 4: transportation unit
- 22: regulator valve
- 25: hydrogen concentration detector
- 31: cooler
- 40: hydrogen production system
- 41: reformer unit
- 42: water-gas shift reaction unit
- 43: methanation reaction unit
- 44: carbon dioxide separation unit
- L6: residual gas circulation line
- L7: gas expulsion line

## Claims

1. A system for hydrogenating an aromatic compound, comprising:
a hydrogenation reaction unit for producing a hydrogenated aromatic compound by adding hydrogen to an aromatic compound via a hydrogenation reaction;
a separation unit for separating the hydrogenated aromatic compound from a product of the hydrogenation reaction unit; and
a transportation unit for circulating at least a part of a residual component remaining in the separation unit after separating the hydrogenated aromatic compound therefrom to the hydrogenation reaction unit;
wherein the hydrogen supplied to the hydrogenation reaction unit consists of diluted hydrogen diluted by a dilution compound having a higher molar specific heat than nitrogen, and the dilution compound includes a component circulated to the hydrogenation reaction unit as the residual component.

2. The system for hydrogenating an aromatic compound according to claim 1, wherein the dilution compound in the diluted hydrogen is in a concentration equal to or greater than 10 vol% and less than 30 vol%.

3. The system for hydrogenating an aromatic compound according to claim 1 or 2, further comprising a circulation line for transporting the residual component to the hydrogenation reaction unit, wherein the circulation line is connected to an expulsion line for expelling a part of the residual component to outside.

4. The system for hydrogenating an aromatic compound according to any one of claims 1 to 3, wherein the dilution compound includes methane, and
the system further comprises a reformer unit for producing reformer gas containing hydrogen and carbon monoxide from the methane via a steam reforming process, and
a methanation reaction unit for producing methane from the carbon monoxide produced by the reformer unit via a methanation reaction,
the hydrogen produced by the reformer unit and the methane produced by the methanation reaction unit being supplied to the hydrogenation reaction unit at least as a part of the diluted hydrogen.

5. A method for hydrogenating an aromatic compound, comprising:
a hydrogenation reaction step for producing a hydrogenated aromatic compound by adding hydrogen to an aromatic compound via a hydrogenation reaction,
a separation step for separating the hydrogenated aromatic compound from a product of the hydrogenation reaction step; and
a transportation step for circulating at least a part of a residual component remaining after the separation step separating the hydrogenated aromatic compound therefrom for use in the hydrogenation reaction step;
wherein the hydrogen supplied for the hydrogenation reaction step consists of diluted hydrogen diluted by a dilution compound, and
the dilution compound includes a component circulated to the hydrogenation reaction step as the residual component.
